# EUROPEAN PATENT APPLICATION

(11) **EP 1 080 729 A1**
(43) Date of publication of application: **07.03.2001**
(21) Application number: 00909635.5
(22) Date of filing: 15.03.2000
(51) Int. Cl.: A61K 45/00, A61K 31/557, A61K 31/41, A61P 43/00, A61P 15/00

(54) **CERVICAL RIPENING AGENT AND CERVICAL RIPENING METHOD**

(30) Priority: 16.03.1999 JP 6952499
(71) Applicant: TORAY INDUSTRIES, INC., Tokyo 103-8666 (JP)
(72) Inventor: OCHI, Yasuo, Kamakura-shi, Kanagawa 248-0036 (JP); HAYASHI, Ryoji, Fujisawa-shi, Kanagawa 251-0033 (JP); ISOGAYA, Masafumi, Yokohama-shi, Kanagawa 245-0016 (JP)
(74) Representative: Kador & Partner
(86) International application number: JP0001557
(87) International publication number: WO0054809

(57) **Abstract**

The present invention provides a cervical ripening agent comprising a prostaglandin EP4 receptor ligand as an effective ingredient, wherein the cervical ripening agent has an excellent cervical ripening action with little adverse effects by allowing it to selectively act on the prostaglandin EP4 receptor.

## Description

### Technical Field

The present invention relates to a cervical ripening agent, and to a new application of a prostaglandin EP4 receptor ligand.

### Background Art

It is thought to be necessary for delivery to make the cervical canal flexible in order to sufficiently expand it for delivery of a fetus, as well as to contract the myometrium as the smooth muscle of the uterus. The cervical canal remains to be tightly closed until the latter stage of pregnancy for continuation of pregnancy, in order to prevent abortion and premature delivery. However, the cervical canal starts to soften for delivery of the fetus after 36 weeks of pregnancy, along with shrinkage of the length of the cervical canal (effacement). This change is termed as cervical ripening. Cervical ripening is a crucial factor for determining delivery process, and this ripening should be normally developed for a baby to be safely delivered. Accordingly, sufficient cervical ripening is inevitable for safe delivery as a preparative step for the delivery, before the myometrium starts to contract.

The number of women who experience delivery in their twenties is decreasing, while the number of women who experience delivery in their thirties or forties is increasing today. It is speculated that the number of women who experience delivery in their twenties may be more decreased, and the number of women who experience delivery in their thirties or forties may be more increased after a decade. The greatest problem in the field of obstetrics and gynecology for the women experiencing delivery in their advanced ages is rigidity of the soft birth canal as well as insufficient cervical ripening. When the degree of cervical ripening is low, the opening of the uterus should be mechanically expanded, or a cervical ripening agent should be administered in order to soften the cervical canal.

Dehydroepiandrosterone sulfate (abbreviated as DHAS hereinafter) is a well known agent for accelerating cervical ripening [Mochizuki, M. et. al., Acta. Obstet. Gynecol. Scand., 57, 397-401 (1978)] that is clinically widely used at present. However, it is a problem that DHAS requires a relatively long period of administration.

It is also known in the art that prostaglandin E₂ (abbreviated as PGE₂ hereinafter) produced as an in vivo metabolite of arachidonic acid has a strong cervical ripening action [Rix, P. et. al., Acta. Obstet. Gynecol. Scand., 75, 45-47(1996)]. However, the physiological functions of PGE₂ involves not only the cervical ripening action, but also many actions such as oxytocic action, inhibition of gastric acid secretion, stimulation of peristalsis of the digestive tract and pyrogenic action. Accordingly, the actions as described above causes adverse effects, when PGE₂ is used for accelerating cervical ripening. In particular, the major adverse effects comprise the oxytocic action, involving such possibilities as causing excess contraction of the uterus or hysterorrhexis when administered to pregnant women.

According to the recent advance of investigations, the receptor for binding PGE₂ can be classifies into four receptor subtypes named as EP1, EP2, EP3 and EP4 [Coleman, R. A. et. al., Pharmacol. Rev., 46, 205-229 (1994)]. It has been made clear that each subtype is related to different physiological functions. For example, the oxytocic action of PGE₂ has been known to be induced by binding of PGE₂ to the EP3 receptor. Compounds that are reported to selectively bind to the EP4 receptor include derivatives of 3,7-dithiaprostanoic acid [Japanese Unexamined Patent Application Publication No. 10-265454, Japanese Unexamined Patent Application Publication No. 2000-1472] and azole derivatives [WO98/55468]. These compounds are disclosed to be effective for immunological diseases.

Accordingly, the object of the present invention is to provide an excellent cervical ripening accelerator that is able to sufficiently ripen the cervical canal with little adverse effects, and a method for cervical ripening.

### Disclosure of Invention

The present invention provides a cervical ripening agent containing a prostaglandin EP4 receptor ligand as an effective ingredient, and a method for cervical ripening.

### Brief Description of the Drawings

Fig. 1 shows the change of the maximum tension measured by extending the cervical canal after administering the compound (A) in the vagina of a guinea pig.
Fig. 2 shows the change of the slope of tension measured by extending the cervical canal after administering the compound (A) in the vagina of a guinea pig.

### Best Mode for Carrying Out the Invention

The prostaglandin EP4 receptor ligand of the present invention is a compound that binds to a EP4 receptor, and includes an agonist and antagonist for the receptor.

Examples of the prostaglandin EP4 receptor ligand include 3,7-dithiaprostanoic acid derivatives and azole derivatives. Concretely they include 3,7-dithiaprostanoic acid derivatives represented by the following general formula (I): 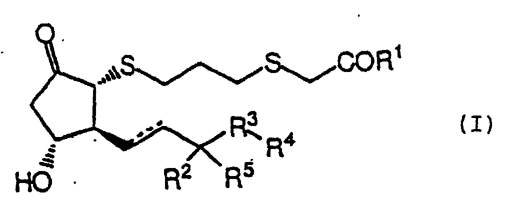 [in the formula, R¹ denotes hydroxy, an alkyloxy group with a carbon number of 1 to 6, or a NR⁶R⁷ group (in the formula, R⁶ and R⁷ independently represent hydrogen or an alkyl group with a carbon number of 1 to 6), R² denotes hydrogen or hydroxy, R³ denotes a single bond or an alkylene group with a carbon number of 1 to 6, R⁴ denotes
(1) an alkyl group with a carbon number of 1 to 8, an alkenyl group with a carbon number of 2 to 8, or an alkynyl group with a carbon number of 2 to 8,
(2) an alkyl group with a carbon number of 1 to 8, an alkenyl group with a carbon number of 2 to 8, or an alkynyl group with a carbon number of 2 to 8, which is substituted with one to three alkyloxy groups with carbon number of 1 to 6 or halogen,
(3) an alkyl group with a carbon number of 1 to 8, an alkenyl group with a carbon number of 2 to 8, or an alkynyl group with carbon number of 2 to 8, which is substituted with phenyl or a cycloalkyl group with a carbon number of 3 to 7,
(4) phenyl, phenyloxy, a cycloalkyl group with a carbon number of 3 to 7, or a cycloalkyloxy group with a carbon number of 3 to 7,
(5) a furyl, furyloxy, thienyl, thienyloxy, naphthyl, naphthyloxy, phthalanyl or phthalanyloxy group,
(6) phenyl, phenyloxy, a cycloalkyl group with a carbon number of 3 to 7, or a cycloalkyloxy group with a carbon number of 3 to 7 substituted with the following one to three groups:
   an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, or hydroxy); or
(7) furyl, furyloxy, thienyl, thienyloxy, naphthyl, naphthyloxy, phthalanyl or phthalanyloxy substituted with the following one to three groups:
   an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, and hydroxy);
   R⁵ denotes hydrogen or an alkyl group with a carbon number of 1 to 6, and
   the mark
      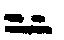 denotes a double bond or a single bond, wherein the alkylene group with a carbon number 1 to 6 represented by R³ may be substituted with one hydroxy when R² denotes hydrogen.], a mixture with a 8-epi compound as an equilibrium compound thereof, or a pharmaceutically acceptable salt thereof or a cyclodextrin clathrate compound thereof; and azole derivatives represented by the following general formula (II); 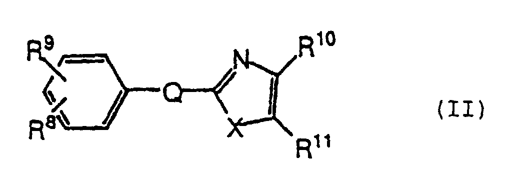 [in the formula, R⁸ denotes;
      (1) an alkyl group with a carbon number of 1 to 6 substituted with hydroxy, a protected carboxyl group or carboxy,
      (2) carboxy,
      (3) a protected carboxyl group,
      (4) carbamoyl,
      (5) a heterocyclic group,
      (6) cyano,
      (7) a haloalkylsulfonyloxy group with a carbon number of 1 to 6,
      (8) an alkyloxy group substituted with hydroxy or carbamoyl,
      (9) an aryl substituted with carboxy, a protected carboxyl group, carbamoyl or a heterocyclic group, or
      (10) an amino group which may be substituted with a protected carboxyl group or an alkylsulfonyl group with a carbon numbers of 1 to 6,
   R⁹ denotes hydrogen or an alkyl group with a carbon number of 1 to 6, R¹⁰ denotes an aryl group which may be substituted with halogen, and R¹¹ denotes an aryl group which may be substituted with halogen, Q represents; 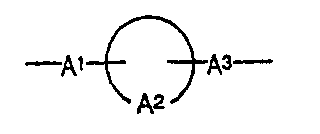 (wherein -A¹- denotes a single bond or an alkylene group with a carbon number of 1 to 6; 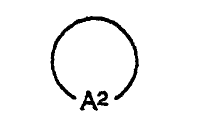 denotes a cycloalkene group with a carbon number of 5 to 9, a cycloalkane group with a carbon number of 3 to 9, a bicycloalkene with a carbon number of 6 to 9, or a bicycloalkane with a carbon number of 5 to 9, and -A³-denotes a single bond or an alkylene with a carbon number of 1 to 6), and X denotes O, NH or S] or a pharmaceutically acceptable salt thereof.

The heterocyclic group as used herein denotes a group comprising a saturated or unsaturated, monocyclic or polycyclic group in which at least one of hetero-atoms is selected from nitrogen, sulfur or oxygen. The protected carboxyl group denotes (1) an alkylester with a carbon number of 1 to 6, (2) an alkenyl ester with a carbon number of 2 to 6, (3) an alkynyl ester with a carbon number of 2 to 6, (4) an arylalkylester which may be substituted with an alkyl group with a carbon number of 1 to 6, an alkyloxy group with carbon number of 1 to 6, phenyl, nitro or halogen, or (5) an arylester which may be substituted with an alkyl group with carbon number of 1 to 6, an alkyloxy group with a carbon number of 1 to 6, phenyl, nitro or halogen, wherein the aryl group denotes phenyl or naphthyl which may be substituted with an alkyl group with a carbon number of 1 to 6.

Examples of the pharmaceutically acceptable salts include an alkali metal salt such as a sodium or potassium salt, an alkali earth metal salt such as a calcium or magnesium salts, an amine salt such as a methylamine, dimethylamine, trimethylamine, methylpiperidine, ethanolamine, diethanolamine, and triethanolamine salts, an ammonium salt, a basic amino acid salt, an organic acid salt such as acetic acid or maleic acid salts, an inorganic acid salt such as hydrochloric acid or sulfuric acid salt, or an acidic amino acid salt.

While one or several kinds of the prostaglandin EP4 receptor ligands or their salts may be directly administer in the vagina or uterus as the cervical ripening agent according to the present invention, they may be administered by adding conventional excipients and stabilizers for formulation of the medicine. Such additives include animal oils, plant oils, paraffin, gum arabic, saccharides such as starch, lactose, sucrose, glucose, dextrin and mannitol, inorganic salts such as calcium carbonate and calcium sulfate, organic acid salts such as sodium citrate, sodium lactate and magnesium stearate, water soluble polymers such as methyl cellulose, gelatin, polyethylene glycol, polyvinyl alcohol, polyvinyl pyrrolidone, hydroxyethyl cellulose and hydroxypropyl cellulose, alcohols such as ethanol, glycerin, propylene glycol and sorbitor, and surfactants such as sorbitan fatty acid ester, polyoxyethylene sorbitan fatty acid ester, polyoxyethylene fatty acid ester and glycerin fatty acid ester.

The cervical ripening agent according to the present invention may be prepared as various formulations, including conventional preparations of a liquid preparation as a vaginal epipastic, a semi-solid preparation such as an ointment, a cream or a gel, and a solid preparation such as a vaginal tablet, a vaginal capsule, a pessary, and a vaginal suppository for the vagina. The agent may be also used as subcutaneous, intravenous and local injection medicines. The agent may be also formulated as tablets, powders, granules and capsules for oral administration.

Although the dosage of the cervical ripening agent differs depending on symptoms, age, conditions of the uterus and types of the administration agent, it is usually 0.0001 mg to 10000 mg, preferably 0.001 mg to 1000 mg, per one adult, which may be administered at once or in several portions.

The cervical ripening agent is applicable for animals other than human. The cervical ripening agent according to the present invention is able to ripen the cervical canal of the animals other than human, and accelerate delivery of fetus when applied for remedy and treatment during pregnancy.

### [EXAMPLE]

The present invention will be described in detail with reference to the example.

### Example 1

### Cervical ripening action:

The cervical ripening action was investigated with respect to the compound (A) shown below represented by the general formula (I), which is reported to selectively bind to the EP4 receptor. [Japanese Unexamined Patent Application Publication No. 10-265454]

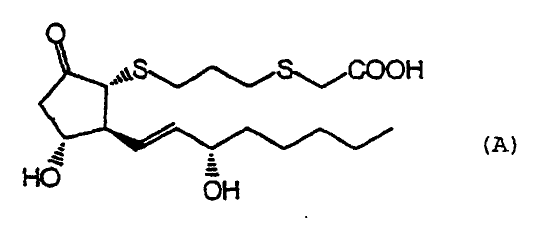

The experiment was carried out using Hartley strain matured guinea pigs (clean, nine weeks of age) with five or six animals in one group. The compound (A) after dissolving in dimethylsulfoxide was diluted with phosphate-buffered saline, and was mixed with 3% hydroxypropyl cellulose immediately before use. The compound (A) (10 µg) or its vehicle was administered in the vagina at 9 and 17 o'clock on the same day, and at 9 o'clock on the day next. The animals were dissected four hours after the final administration, and the cervical canal was extracted. Then, the cervical canal was extended at a speed of 2 mm/min to measure time-dependent changes of the tension generated in the tissue. The maximum tension generated before the tissue is broken was measured, and slope of the tension was calculated by dividing the maximum tension by the extension length before the tension reaches maximum. It is thought that decreases in these values indicate ripening of the cervical canal.

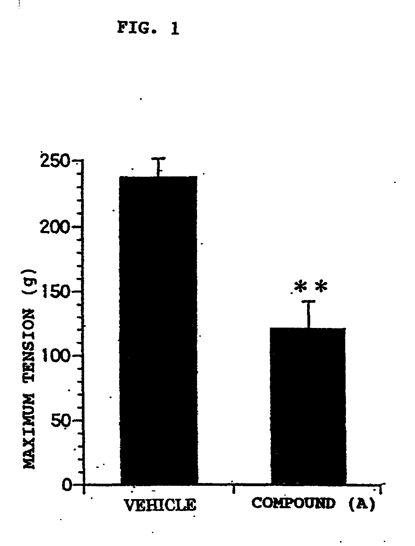

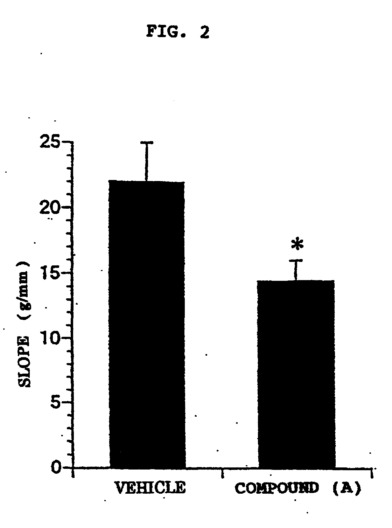

Figs. 1 and 2 show the changes of the maximum tension and slope of the tension induced by administration of the compound (A)(*: P < 0.05, **: P < 0.01, t - test, comparison with the group that received the vehicle). The figures clearly show that the compound (A) made the maximum tension and slope decrease as compared with the results in the groups who received the vehicle.

It was shown from the results above that the compound (A) has an excellent cervical ripening action.

### Industrial Applicability

The cervical ripening agent according to the present invention is useful with little adverse effects, since it has an excellent ripening action on the cervical canal by selectively acting on the prostaglandin EP4 receptor.

## Claims

1. A cervical ripening agent comprising a prostaglandin EP4 receptor ligand as an effective ingredient.

2. A cervical ripening agent according to Claim 1, wherein the prostaglandin EP4 receptor ligand comprises 3,7-dithiaprostanoic acid derivatives or azole derivatives.

3. A cervical ripening agent according to Claim 1, wherein the prostaglandin EP4 receptor ligand is a 3,7-dithiaprostanoic acid derivative represented by the following general formula (I); 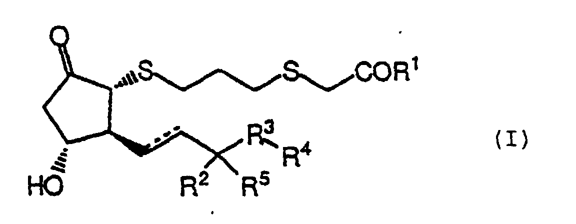 [in the formula, R¹ denotes hydroxy, an alkyloxy group with a carbon number of 1 to 6, or a NR⁶R⁷ group (in the formula, R⁶ and R⁷ independently represent hydrogen or an alkyl group with a carbon number of 1 to 6), R² denotes hydrogen or hydroxy, R³ denotes a single bond or an alkylene group with a carbon number of 1 to 6, R⁴ denotes
(1) an alkyl group with a carbon number of 1 to 8, an alkenyl group with a carbon number of 2 to 8, or an alkynyl group with a carbon number of 2 to 8,
(2) an alkyl group with a carbon number of 1 to 8, an alkenyl group with a carbon number of 2 to 8, or an alkynyl group with a carbon number of 2 to 8, which is substituted with one to three alkyloxy groups with carbon number of 1 to 6 or halogen,
(3) an alkyl group with a carbon number of 1 to 8, an alkenyl group with a carbon number of 2 to 8, or an alkynyl group with carbon number of 2 to 8, which is substituted with phenyl or a cycloalkyl group with a carbon number of 3 to 7,
(4) phenyl, phenyloxy, a cycloalkyl group with a carbon number of 3 to 7, or a cycloalkyloxy group with a carbon number of 3 to 7,
(5) a furyl, furyloxy, thienyl, thienyloxy, naphthyl, naphthyloxy, phthalanyl or phthalanyloxy group,
(6) phenyl, phenyloxy, a cycloalkyl group with a carbon number of 3 to 7, or a cycloalkyloxy group with a carbon number of 3 to 7 substituted with the following one to three groups:
an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, or hydroxy); or
(7) furyl, furyloxy, thienyl, thienyloxy, naphthyl, naphthyloxy, phthalanyl or phthalanyloxy substituted with the following one to three groups:
an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, and hydroxy);
R⁵ denotes hydrogen or an alkyl group with a carbon number of 1 to 6, and
the mark
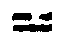 denotes a double bond or a single bond, wherein the alkylene group with a carbon number 1 to 6 represented by R³ may be substituted with one hydroxy when R² denotes hydrogen.], a mixture with a 8-epi compound as an equilibrium compound thereof, or a pharmaceutically acceptable salt thereof or a cyclodextrin clathrate compound thereof.

4. A cervical ripening agent according to Claim 1, wherein the prostaglandin EP4 receptor ligand is an azole derivative represented by the following general formula (II); 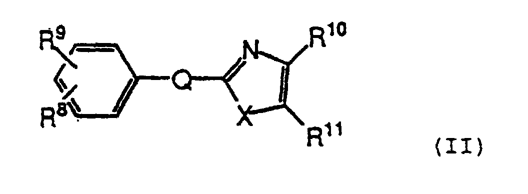
[in the formula, R⁸ denotes;
(1) an alkyl group with a carbon number of 1 to 6 substituted with hydroxy, a protected carboxyl group or carboxy,
(2) carboxy,
(3) a protected carboxyl group,
(4) carbamoyl,
(5) a heterocyclic group,
(6) cyano,
(7) a haloalkylsulfonyloxy group with a carbon number of 1 to 6,
(8) an alkyloxy group substituted with hydroxy or carbamoyl,
(9) an aryl substituted with carboxy, a protected carboxyl group, carbamoyl or a heterocyclic group, or
(10) an amino group which may be substituted with a protected carboxyl group or an alkylsulfonyl group with a carbon numbers of 1 to 6,
R⁹ denotes hydrogen or an alkyl group with a carbon number of 1 to 6, R¹° denotes an aryl group which may be substituted with halogen, and R¹¹ denotes an aryl group which may be substituted with halogen, Q represents; 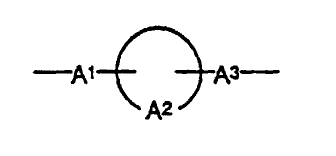 (wherein -A¹- denotes a single bond or an alkylene group with a carbon number of 1 to 6; 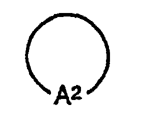 denotes a cycloalkene group with a carbon number of 5 to 9, a cycloalkane group with a carbon number of 3 to 9, a bicycloalkene with a carbon number of 6 to 9, or a bicycloalkane with a carbon number of 5 to 9, and -A³-denotes a single bond or an alkylene with a carbon number of 1 to 6), and X denotes O, NH or S]or a pharmaceutically acceptable salt thereof.

5. A method for cervical ripening, wherein an effective dose of a prostaglandin EP4 receptor ligand is administered to human or animals.

6. A method for cervical ripening according to Claim 5, wherein the prostaglandin EP4 receptor ligand is a 3,7-dithiaprostanoic acid derivative or an azole derivative.

7. A method for cervical ripening according to Claim 5, wherein the prostaglandin EP4 receptor ligand is a 3,7-dithiaprostanoic acid derivative represented by the following general formula (I): 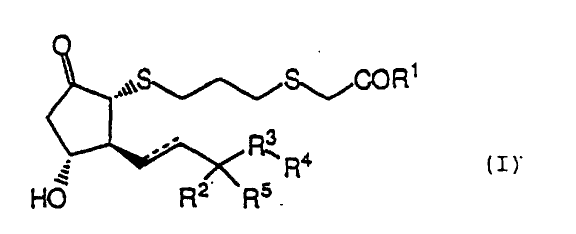 [in the formula, R¹ denotes hydroxy, an alkyloxy group with a carbon number of 1 to 6, or a NR⁶R⁷ group (in the formula, R⁶ and R⁷ independently represent hydrogen or an alkyl group with a carbon number of 1 to 6), R² denotes hydrogen or hydroxy, R³ denotes a single bond or an alkylene group with a carbon number of 1 to 6, R⁴ denotes
(1) an alkyl group with a carbon number of 1 to 8, an alkenyl group with a carbon number of 2 to 8, or an alkynyl group with a carbon number of 2 to 8,
(2) an alkyl group with a carbon number of 1 to 8, an alkenyl group with a carbon number of 2 to 8, or an alkynyl group with a carbon number of 2 to 8, which is substituted with one to three alkyloxy groups with carbon number of 1 to 6 or halogen,
(3) an alkyl group with a carbon number of 1 to 8, an alkenyl group with a carbon number of 2 to 8, or an alkynyl group with carbon number of 2 to 8, which is substituted with phenyl or a cycloalkyl group with a carbon number of 3 to 7,
(4) phenyl, phenyloxy, a cycloalkyl group with a carbon number of 3 to 7, or a cycloalkyloxy group with a carbon number of 3 to 7,
(5) a furyl, furyloxy, thienyl, thienyloxy, naphthyl, naphthyloxy, phthalanyl or phthalanyloxy group,
(6) phenyl, phenyloxy, a cycloalkyl group with a carbon number of 3 to 7, or a cycloalkyloxy group with a carbon number of 3 to 7 substituted with the following one to three groups:
an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, or hydroxy); or
(7) furyl, furyloxy, thienyl, thienyloxy, naphthyl, naphthyloxy, phthalanyl or phthalanyloxy substituted with the following one to three groups:
an alkyl group with a carbon number of 1 to 6, an alkenyl group with a carbon number of 2 to 6, an alkynyl group with a carbon number of 2 to 6, an alkyloxy group with a carbon number of 1 to 6, an alkyl group with a carbon number of 1 to 6 substituted with alkyloxy groups with a carbon number of 1 to 6, an alkyloxy group with a carbon number of 1 to 6 substituted with alkyloxy groups with a carbon number of 1 to 6, an alkyl group with a carbon number of 1 to 6 substituted with alkenyloxy groups with a carbon number of 2 to 6, an alkyl group with a carbon number of 1 to 6 substituted with one to three hydroxy, an alkyl group with a carbon number of 1 to 6 substituted with one to three halogen, an alkylthio group with a carbon number of 1 to 6, an alkyl group with a carbon number of 1 to 6 substituted with alkylthio groups with a carbon number of 1 to 6, an alkyloxy group with a carbon number of 1 to 6 substituted with alkylthio groups with a carbon number of 1 to 6, an alkyl group with a carbon number of 1 to 6 substituted with alkenylthio groups with a carbon number of 2 to 6, an alkylsulfonyl group with a carbon number of 1 to 6, halogen, a trihalomethyl group, cyano, nitro, amino, hydroxy, a cycloalkyl group with a carbon number of 3 to 7, a cycloalkyloxy group with a carbon number of 3 to 7, an alkyl group with a carbon number of 1 to 6 substituted with cycloalkyl groups with a carbon number of 3 to 7, an alkyl group with a carbon number of 1 to 6 substituted with cycloalkyloxy groups with a carbon number of 3 to 7, phenyl, phenyloxy, an alkyl group with a carbon number of 1 to 6 substituted with phenyl, an alkenyl group with a carbon number of 2 to 6 substituted with phenyl, an alkynyl group with a carbon number of 2 to 6 substituted with phenyl, an alkyl group with a carbon number of 1 to 6 substituted with phenyloxy, an alkenyl group with a carbon number of 2 to 6 substituted with phenyloxy, an alkynyl group with a carbon number of 2 to 6 substituted with phenyloxy, furyl, furyloxy, an alkyl group with a carbon number of 1 to 6 substituted with furyl, an alkyl group with a carbon number of 1 to 6 substituted with furyloxy, thienyl, thienyloxy, an alkyl group with a carbon number of 1 to 6 substituted with thienyl, or an alkyl group with a carbon number of 1 to 6 substituted with thienyloxy (the phenyl, furyl, thienyl and cycloalkyl group may be substituted with one to three alkyl groups with a carbon number of 1 to 6, alkyloxy groups with a carbon number of 1 to 6, alkyl groups with a carbon number of 1 to 6 substituted with alkyloxy groups with a carbon number of 1 to 6, nitro, halogen, trihalomethyl groups, amino, and hydroxy);
R⁵ denotes hydrogen or an alkyl group with a carbon number of 1 to 6, and
the mark
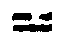 denotes a double bond or a single bond, wherein the alkylene group with a carbon number 1 to 6 represented by R³ may be substituted with one hydroxy when R² denotes hydrogen.], a mixture with a 8-epi compound as an equilibrium compound thereof, or a pharmaceutically acceptable salt thereof or a cyclodextrin clathrate compound thereof.

8. A method for cervical ripening according to Claim 5, wherein the prostaglandin EP4 receptor ligand is an azole derivative represented by the following general formula (II); 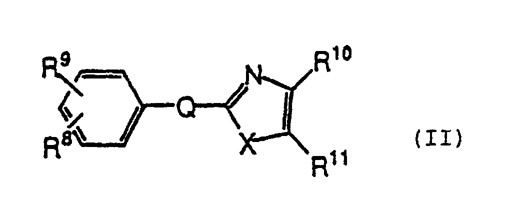
[in the formula, R⁸ denotes;
(1) an alkyl group with a carbon number of 1 to 6 substituted with hydroxy, a protected carboxyl group or carboxy,
(2) carboxy,
(3) a protected carboxyl group,
(4) carbamoyl,
(5) a heterocyclic group,
(6) cyano,
(7) a haloalkylsulfonyloxy group with a carbon number of 1 to 6,
(8) an alkyloxy group substituted with hydroxy or carbamoyl,
(9) an aryl substituted with carboxy, a protected carboxyl group, carbamoyl or a heterocyclic group, or
(10) an amino group which may be substituted with a protected carboxyl group or an alkylsulfonyl group with a carbon numbers of 1 to 6,
R⁹ denotes hydrogen or an alkyl group with a carbon number of 1 to 6, R¹⁰ denotes an aryl group which may be substituted with halogen, and R¹¹ denotes an aryl group which may be substituted with halogen, Q represents; 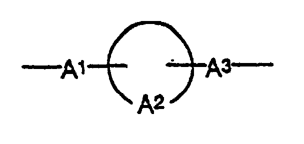 (wherein -A¹- denotes a single bond or an alkylene group with a carbon number of 1 to 6; 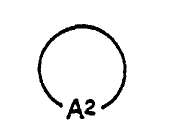 denotes a cycloalkene group with a carbon number of 5 to 9, a cycloalkane group with a carbon number of 3 to 9, a bicycloalkene with a carbon number of 6 to 9, or a bicycloalkane with a carbon number of 5 to 9, and -A³-denotes a single bond or an alkylene with a carbon number of 1 to 6), and X denotes O, NH or S]or a pharmaceutically acceptable salt thereof.
